# EUROPÄISCHE PATENTSCHRIFT

(11) **EP 2 494 935 B1**
(45) Veröffentlichungstag und Bekanntmachung des Hinweises auf die Patenterteilung: **30.04.2014**
(21) Anmeldenummer: 12157328.1
(22) Anmeldetag: 28.02.2012
(51) Int. Cl.: A61B 18/20, A61N 5/06

(54) **Vorrichtung und Verfahren zur kosmetisch-ästhetischen Hautbehandlung**
Method and device for cosmetic-aesthetic skin treatment
Dispositif et procédé de traitement dermatologique esthétique et cosmétique

(30) Priorität: 03.03.2011 DE 102011005069
(43) Veröffentlichungstag der Anmeldung: 05.09.2012
(73) Patentinhaber: Beauty Lumis GmbH, 80995 München (DE)
(72) Erfinder: Frommer, Adelbert, Munchen 80995 (DE); Wittenberg, Dieter, 13581 Berlin (DE)
(74) Vertreter: Klunker . Schmitt-Nilson . Hirsch

(56) Entgegenhaltungen:
- WO-A1-01/87176
- WO-A1-99/39624
- US-A1- 2002 088 779

## Beschreibung

Die Erfindung betrifft eine Vorrichtung zur kosmetisch-ästhetischen Hautbehandlung sowie ein entsprechendes Verfahren zur kosmetisch-ästhetischen Hautbehandlung. Insbesondere betrifft die Erfindung eine Vorrichtung zur kosmetisch-ästhetischen Hautbehandlung mit einem über einen Trigger-Eingang triggerbaren Impuls-Laser, dessen optischer Ausgang so eingerichtet ist, dass er auf einen Hautbereich gerichtet werden kann, und mit einer Einrichtung zum Triggern des Impuls-Lasers mit einem Ausgang, der mit dem Trigger-Eingang des Impulslasers verbunden ist. In entsprechender Weise betrifft die Erfindung insbesondere ebenfalls ein Verfahren zur kosmetisch-ästhetischen Hautbehandlung mit einem Schritt des Auftragens einer kurzkettigen Hyaluronsäure-Zubereitung auf einen Hautbereich und einem weiteren Schritt des Einwirkens von gepulstem Laserlicht auf den Hautbereich.

Es ist allgemein bekannt, dass die menschliche Haut im Zuge ihrer Alterung faltig wird. Die Elastizität der Haut geht zurück, wenn es bei zunehmendem Altern von Elastinfasern zur Ablagerung von Fettsäuren, Aminosären und Kalisalzen kommt, die dann die Wasserhülle der Fasern derart ändern, so dass diese ihre Elastizität nach und nach verlieren. Dies führt im Endeffekt zu einem ästhetischen Problem, das mit kosmetischen Vorrichtungen und Verfahren angegangen werden kann.

Insbesondere sind zur Hautfaltigkeitsverminderung kosmetisch-ästhetische Hautbehandlungsverfahren bekannt, bei denen Hyaluronsäurepräparate eingesetzt werden.

Hyaluronsäure ist ein Bestandteil der extrazellulären Matrix (EZM) von Wirbeltieren. Es liegt in vielerlei Geweben als langkettiges, lineares Polysaccharid vor und erfüllt viele Funktionen, wobei die zahlreichen verschiedenen chemisch-physikalischen Eigenschaften dieser Verbindungen eine Rolle spielen. Die einzelnen Ketten können eine molare Masse von mehreren Millionen atomaren Masseneinheiten erreichen. Die Hyaluronsäure verleiht dem Bindegewebe hohe Druck- und Formelastizität und bildet glatte Oberflächen. In kosmetischen Zubereitungen wie Cremes, Lotionen etc. wird diese Substanz als Feuchte erhaltende Komponente zugesetzt.

Es ist bekannt, Hyaluronsäurepräparate zur Faltenunterspritzung, zum Modellieren der Lippen, zur Hautauffrischung und auch zum Aufbau von Gesichtskonturen zu verwenden. Je nach Stabilisierung der Hyaluronsäure bleibt der so gewonnene Effekt 6 bis 12 Monate erhalten. Unter Implantaten werden in der kosmetischen Dermatologie Materialien zur Unterspritzung von Falten oder Auffüllung subcutaner Gewebedefekte verstanden. Andere gebräuchliche Bezeichnungen sind Filler, Injectables oder Augmentationsmaterialien. Sie zählen zu den gering invasiven Behandlungsmöglichkeiten gegen Hautalterung. Es ist bekannt, eine Unterspritzung von Falten mit Hyaluronsäurepräparaten in Linear- oder Punkttechnik durchzuführen, feinen perioralen Falten in die obere Dermis, bei tieferen Falten in die tiefe Dermis. Bei höher viskösen Hyaluronsäurepräparaten ist es erforderlich auf dickere Injektionsnadeln zurückzugreifen, was beim Patienten größere Schmerzen verursachen kann. Die Injektionen setzte eine geübte Technik voraus, da abhängig vom Hauttyp (Koriumdicke) Probleme auftreten können.

Aufgrund dieser Probleme kommen neben der intracutanen Anwendung von Hyaluronsäure auch Verfahren zu epikutanen Applikation von Uroniden in Betracht. Dabei steht die große Molmasse der Hyaluronsäure einer Penetration selbst in die obersten Anteile des Stratum corneum entgegen. Es muss damit gerechnet werden, dass sich die Substanz lediglich in bestehende Spalten des Stratum corneum verteilt. Dadurch ist zwar durchaus ein gewisser kosmetischer Effekt im Sinne einer Glättung der Hautoberfläche erzielbar, aber eine funktionale Beeinflussung hinsichtlich der Barrierefunktion des Stratum corneum oder gar eine Einflussnahme auf vitale Epidermnisschichten ist kaum zu erwarten. Uronide besitzen hingegen eine deutlich geringere Molmasse und deshalb bessere Voraussetzungen für die Penetration.

Bei einem anderen bekannten Verfahren werden Hyaluronsäure-Moleküle beispielsweise mit UV-Licht aufgebrochen, in die Haut einmassiert und anschließend mit einem Infrarot-Kaltlichtflächenlaser behandelt, wodurch die einmassierten Hyaluronsäure-Bruchstücke in der Haut wieder zu Makromolekülen reagieren. Dadurch kann die Wirkung von äußerlich applizierter Hyaluronsäure auf mehrere Wochen verlängert werden.

Insbesondere ist es auch bekannt, ein Hyaloronat in das Hautgewebe einzubringen und mittels Photonenenergie (Laserlicht) so zu energetisieren, dass die Eigenschaft des Markromoleküls Hyaloron, in großen Mengen Wasser quellen zu können, einerseits dazu benutzt wird, die reversiblen Hautfalten infolge der Volumenzunahme beim Quellvorgang nach außen zu drücken, wodurch sich die Falten glätten:
Der aus dem Stand der Technik bekannte invasive Eintrag von Hyaluronsäure durch Injektionen kann mit Nebenwirkungen verbunden sein und wird oft als belastend empfunden. Demgegenüber besteht bei dem ebenfalls aus dem Stand der Technik bekannten äußerlichem Auftrag von Hyaluronsäure-Präparaten das Problem, die Hyaluronsäure-Moleküle in die tieferen Hautschichten einzubringen. Das Einmassieren kurzkettiger Hyaluronsäure-Präparate und nachfolgende Behandlung mit Laserlicht vermeidet die Nachteile invasiver Verfahren, ist jedoch noch offen für Verbesserungen, die geeignet sind, die Aufnahme der Hyaluronsäure in tiefere Hautschichten zu erleichtern.

Die WO 01/87176 A1 lehrt eine Vorrichtung gemäß Oberbegriff des Anspruchs 1.

Zum Stand der Technik wird noch auf folgende Druckschriften aus der Nicht-Patentliteratur hingewiesen: Konrad Kunsch, Steffen Kunsch, Der Mensch in Zahlen, Spektrum Akademischer Verlag, 3. Auflage 2007. Harvey Lodish, David Baltimore, Arnold Berk, S.Lawrence Zipursky, Paul Matsudaira, James Darnell, Molekulare Zellbiologie, Walter de Gruyter, 2. Auflage, Berlin New York 1996. Thomas D. Pollard, William C. Earnshaw, Jennifer Lippincott-Schwartz, Cell Biology, Spektrum Akademischer Verlag, Second Edition, 2007. Alberts, Johnson, Lewis, Raff, Roberts, Walter, Molekularbiologie der Zelle, 4. Auflage, 2004 WILEY-VCH Verlag GmbH & Co. KGaA, Weinheim. W.Wohlrab, R.H.H. Neubert, J. Wohlrab, Hyaluronsäure und Haut, Shaker Verlag, Aachen 2004. P. Prehm, Hyaluronan, der Gewebe-Expander, Biospektrum 05.08., S. 473-475. Bernhard Michel, Paul Holcomb, Simulation der Lichtstreuung in menschlicher Haut, Photonik 3/2007. Andrei P. Sommer, Dan Zhu, Tim Scharnweber, Extraordinary Anticancer Effect of Green Tea and Red Light, Photomedicine and Laser Surgery, Vol. 28, Number 3, 2010. Bernd Schöller, Pulsoximetrie-Fibel, MCC GmbH 1994 (www.mcc-med.de) E. Hering, R. Martin, Photonik, Springer-Verlag, Berlin, Heidelberg, 2006.

Aufgabe der Erfindung ist es, eine Vorrichtung und ein Verfahren zur kosmetisch-ästhetischen Hautbehandlung anzugeben, mit der der kosmetische Behandlungserfolg verbessert werden kann.

Diese Aufgabe wird erfindungsgemäß gelöst durch eine Vorrichtung zur kosmetisch-ästhetischen Hautbehandlung mit einem über einen Trigger-Eingang triggerbaren Impuls-Laser, dessen optischer Ausgang so eingerichtet ist, dass er auf einen Hautbereich einer kosmetisch zu behandelnden Person gerichtet werden kann, und mit einer Einrichtung zum Triggern des Impuls-Lasers mit einem Ausgang, der mit dem Trigger-Eingang des Impulslasers verbunden ist, der gemäß den im Anspruch 1 angegebenen Merkmalen weitergebildet ist. Ferner wird diese Aufgabe erfindungsgemäß gelöst durch ein Verfahren zur kosmetisch-ästhetischen Hautbehandlung mit einem Schritt des Auftragens einer kurzkettigen Hyaluronsäure-Zubereitung auf einen Hautbereich einer kosmetisch zu behandelnden Person und einem weiteren Schritt des Einwirkens von gepulstem Laserlicht auf den Hautbereich, das gemäß den im Anspruch 9 angegebenen Merkmalen weitergebildet ist. Schließlich wird diese Aufgabe gelöst durch Verwendung einer Vorrichtung nach einem der Ansprüche 1 bis 8 zur Durchführung eines Verfahrens nach einem der Ansprüche 9 bis 15.

Es ist experimentell nachgewiesen worden, dass in Vitro gepulstes Laserlicht den Stoffwechsel von Zellen derart aktiviert, dass diese zur Aufnahme von kleinen Molekülen (zum Beispiel Nährstoffe und Antioxidantien) gezwungen werden können.

Bei dem überwiegenden Teil der hydrophilen Gesamtflächen in Zellen (Makromoleküle, Organellen) bildet eingeschlossenes Wasser Grenzwasser-Flächen (sehr dünne Flächen, deren Dicke etwa dem Durchmesser der Wassermoleküle entspricht).

Durch Untersuchungen wurde festgestellt, dass dann, wenn kohärentes Licht bestimmter Wellenlänge, Frequenz und Energie auf diese Grenzwasserflächen trifft, sich augenblicklich deren Fließfähigkeit verändert, während analog dazu die Dichte der Grenzwassersschicht abnimmt, d.h. die Wassermoleküle entfernen sich voneinander. Die betroffenen Zellen reagieren hierauf reflexartig und versuchen, den alten Molekülabstand zu halten, indem sie sich zusammenziehen. Dabei stoßen sie zwangsläufig einen Teil des Cytosols aus. Nachdem die Bestrahlung mit dem Laserlicht beendet ist, dehnen sich die Zellen wiederum reflexartig auf das alte Zellvolumen aus (Widerherstellung des vorherigen Molekülabstandes), wobei das Volumen der vorher ausgestoßenen Zellflüssigkeit wieder in die Zelle eingeschleust wird. Dabei nimmt die Zelle auf, was immer sich in ihrem unmittelbaren Umfeld befindet und was von den Transportkanälen größenmäßig verarbeitet werden kann. Dieser Vorgang wird als transmembraner Diffusionsreflex bezeichnet.

Ein Diffusionsprozess beschreibt einen Vorgang, bei dem beispielsweise Wasser mit all seinen sonstigen Inhaltsstoffen vollständig eine Membran durchdringt. Im Gegensatz dazu durchdringt Wasser bei einem osmotischen Prozess eine Membran vollständig, seine sonstigen Inhaltstoffe verbleiben aber vor der Membran, d.h. der osmotische Prozess trennt in diesem Fall die Inhaltsstoffe vom Wasser. Ein Transmembranprotein ist ein in die Lipidschicht einer Biomembran eingelagertes (integrales) Protein , welches durch die Lipid-Doppelschicht hindurchgeht, wobei Teile von ihm an beiden Seiten hervorragen. Dabei bauen sich die meisten Membranproteine mit einer α - Helix, mehreren α - Helices oder mittels β - Faltblättern in die Membran ein. Transmembrane Diffusion ist also ein Prozess, der bei Zellen für bestimmte Stoffe und unter bestimmten Bedingungen "offene Türen" in den Lipid-Doppelschichten bereitstellt. Man unterscheidet die Transmembranproteine funktionell. Transporter und Kanäle sind essentiell, um u.a. das lonengleichgewicht einer Zelle zu gewährleisten. Enzyme katalysieren wichtige Stoffwechselprozesse, Rezeptoren dienen einer Weiterleitung von Signalen über die Lipidschicht hinweg, Connexide knüpfen direkte Verbindungen zu Zellen.

Durch den transmembranen Diffusionsreflex gerät der Zellverband in Bewegung, d.h. das Pumpen der Zellen lockert verfestigte Strukturen. Zuvor bereitgestellte Nährstoffe werden dadurch und durch die Effekte der Volumenzunahme der zuvor einmassierten Hyaluronsäurezubereitung zu den Zellen transportiert und dort dynamisch verwertet. Das wiederum erhöht die Nachhaltigkeit und führt im Fortschritt der Behandlung - im Rahmen der Regenerationsfähigkeit der Zellen und Gewebe - zu einer konstanten Verbesserung des Gesamtbildes.

Während eines Herzzyklus füllen sich zunächst die Vorhöfe, während gleichzeitig die Kammern das Blut in die Arterien auswerfen. Wenn sich die Kammermuskulatur entspannt, öffnen sich die Segelklappen und das Blut fließt, gesaugt durch den Druckabfall in den Kammern, aus den Vorhöfen in die Kammern. Unterstützt wird dies durch ein Zusammenziehen der Vorhöfe (Vorhofsystole). Es folgt die Kammersystole. Hierbei zieht sich die Kammermuskulatur zusammen, der Druck steigt an, die Segelklappen schließen sich und das Blut kann nur durch die nun geöffneten Taschenklappen in die Arterien ausströmen. Ein Rückfluss des Blutes aus den Arterien während der Entspannungsphase (Diastole) wird durch den Schluss der Taschenklappen verhindert. Die Erfindung steht im Zusammenhang mit der Erkenntnis, dass die Aufnahmebereitschaft der Haut für kurzkettige Hyaluronsäurepräparate mit den einzelnen Phasen des Herzschlagszyklus korrelliert. In der dyastolischen Phase ist diese Aufnahmebereitschaft signifikant höher als in der systolischen Phase.

Das erfindungsgemäße Verfahren ist seiner technischen Beschaffenheit nach kosmetischer Natur, d.h. es dient lediglich dazu, äußerlich am Körper des Menschen zur Erhaltung eines guten Zustandes und Veränderung des Aussehens angewendet zu werden.

Die Erfindung wird nunmehr anhand von Ausführungsbeispielen und unter Bezugnahme auf die beigefügten Fign. näher erläutert.
- Fig. 1: zeigt ein Blockschaltbild einer Vorrichtung zur kosmetisch-ästhetischen Hautbehandlung aus dem Stand der Technik.
- Fig. 2: zeigt ein Blockschaltbild einer erfindungsgemäßen Vorrichtung zur kosmetisch-ästhetischen Hautbehandlung.
- Fig. 3: zeigt ein Signaldiagramm mit Signalverläufen aus der erfindungsgemäßen Lösung.

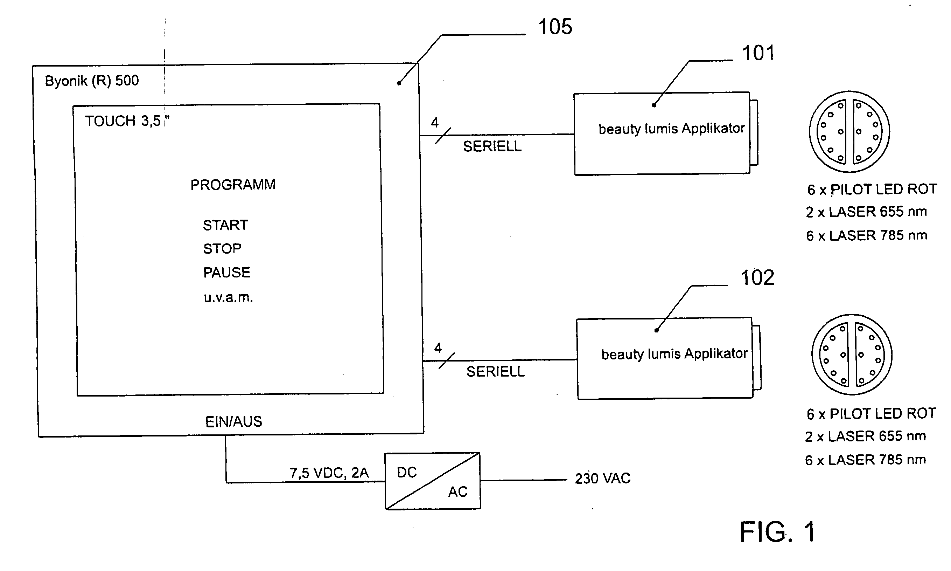

Fig. 1 zeigt ein Blockschaltbild einer exemplarischen Vorrichtung zur kosmetisch-ästhetischen Hautbehandlung aus dem Stand der Technik. Zwei Applikatoren 101, 102 sind mit Laserdioden bestückt, die jeweils auf einen Hautbereich der kosmetisch zu behandelnden Person gerichtet werden können. Jeder der beiden bekannten Applikatoren 101, 102 umfaßt eine Anzahl von rotes Licht emittierenden Leuchtdioden, deren Lichtausstrahlung als solche zwar für die intendierte Hautbehandlung ohne Bedeutung ist, aber den Bereich ausleuchtet, der bei der Aktivierung der Applikatoren mit Laserlicht bestrahlt wird. Auf diese Weise ist es für den Anwender möglich, den zu behandelnden Hautbereich genau zu lokalisieren. Für die Behandlung enthält jeder der beiden Applikatoren 101, 102 eine Anzahl von auf den Hautbereich gerichteten Laserdioden, im vorliegenden Fall 2 Laserdioden mit einem optischen Ausgang bei 655 nm Wellenlänge und 6 Laserdioden mit einem optischen Ausgang bei 785 nm. Die bekannt Vorrichtung ist so dimensioniert, dass die Gesamt-Laserleistung je Applikator 101, 102 bei 500 mW liegt.

Die Ansteuerung der Applikatoren 101, 102 erfolgt durch eine Steuerungseinheit 105. Durch geeignete Bedienelemente (nicht dargestellt) können die Laserdioden in den Applikatoren 101, 102 gesteuert werden.

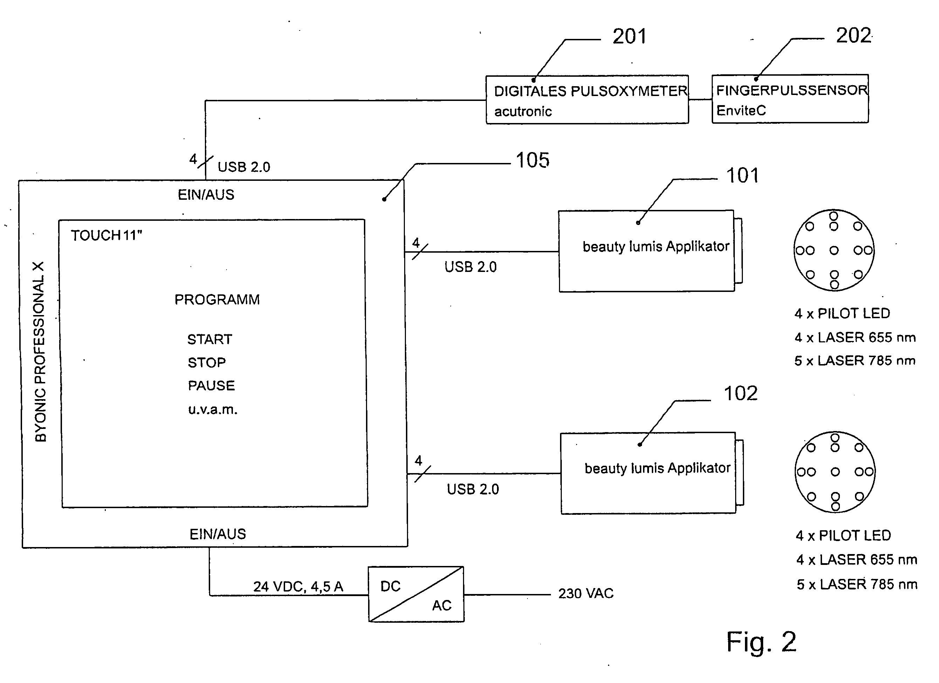

Fig. 2 zeigt ein Blockschaltbild einer erfindungsgemäßen Vorrichtung zur kosmetisch-ästhetischen Hautbehandlung. Die in Fig. 1 mit gleichen Bezugszeichen bezeichneten Teile entsprechen dem aus dem Stand der Technik bekannten System. Gegenüber der aus Fig. 1 bekannten Vorrichtung umfaßt die in Fig. 2 dargestellte Vorrichtung ein Pulsoxymeter 201 mit einem am Körper der kosmetisch zu behandelnden Person zu befestigenden Pulsoxymeter-Sensor 202. Ein Herzschlagssignal-Ausgang des Pulsoxymeters ist mit einem Steuerungseingang der Steuerungseinheit 105 verbunden. In dem erfindungsgemäßen Ausführungsbeispiel aus Fig. 2 steuert die Steuerungseinheit 105 die Laserdioden in den Applikatoren 101, 102 derart an, dass Laserimpulse ausgesandt werden, die mit dem Herzschlag synchronisiert sind. Eine alternative Ausführungsform (nicht dargestellt) umfaßt nur einen Applikator 101.

Die Steuerungseinheit 105 kann sowohl als festverdrahtete Schaltung als auch als programmierbarer Mikrocontroller (beispielsweise AT90USB1287, beziehbar über Atmel Corporation, 2325 Orchard Parkway, San Jose, CA 95131, USA) ausgeführt werden, auf dem ein geeignetes Steuerungsprogramm abläuft, das die Applikatoren auf eine geeignete Art und Weise ansteuert, so dass Laserimpulse ausgesandt werden, die mit dem Herzschlag synchronisiert sind. In einer Ausführungsform werden Laserdioden vom Typ ADL78901TU (785 nm Wellenlänge) und ADL66503TU (655 nm Wellenlänge) verwendet. Beide Typen sind über die Laser Components GmbH, Werner-von-Siemens-Straße 15, 82140 Olching, Deutschland, beziehbar. In einer Ausführungsform werden diese Laserdioden angesteuert über eine integrierte Treiberschaltung vom Typ iC-NZP, beziehbar über die iC-Haus GmbH, Am Kuemmerling 18, 55294 Bodenheim, Deutschland.

Die Pulsoxymetrie ist ein als solches bekanntes Verfahren zur nicht invasiven Ermittlung der arteriellen Sauerstoffsättigung über die Messung der Lichtabsorption bzw. der Lichtremission bei Durchleuchtung der Haut (perkutan). Die Messung erfolgt beispielsweise mit einem Sättigungsaufnehmer (Clip oder Klebesensor) 202 an einem leicht zugänglichen Körperteil, vorzugsweise an einem Finger, Zeh, am Ohrläppchen. Der Sensor hat auf der einen Seite zwei in einem definierten (Infra-)Rot-Bereich leuchtende Lichtquellen (nicht dargestellt), auf der anderen Seite einen Fotosensor (nicht dargestellt). Durch die unterschiedliche Färbung des mit Sauerstoff gesättigten Hämoglobins entsteht für das durchstrahlende Rotlicht eine unterschiedliche Absorption, die der Fotosensor misst. Über den Clip oder Klebesensor kann neben der Sättigung insbesondere auch der Puls in den kleinsten Blutgefäßen (Kapillaren) erfasst werden. Gemessen werden üblicherweise drei Werte, die Absorption des Lichts im 660 nm-Bereich, im 940 nm-Bereich und - zur Tarierung - ohne die Strahlung der Messlichtquellen, nur mit Umgebungslicht. Die unterschiedliche Absorption des Lichtes ergibt eine Differenz, die zur Bestimmung des Herzschlages herangezogen werden kann.

Alternativ ist es aber auch möglich, die Laserbestrahlung durch die Applikatoren 101, 102 auf andere Weise als durch ein Pulsoxymeter mit dem Herzschlag zu synchronisieren. Beispielsweise kann ein modifiziertes Ausführungsbeispiel (nicht dargestellt) vorgesehen werden, bei dem eine Elektrokardiographie-Einrichtung anstelle eines Pulsoxymeters zur Detektion des Herzschlages verwendet wird. Die Elektrokardiographie-Einrichtung detektiert ein Elektrokardiogramm (EKG), d.h. eine Aufzeichnung der Summe der elektrischen Aktivitäten aller Herzmuskelfasern, und wertet diese aus. Elektrokardiographie-Einrichtungen sind dem Fachmann weithin bekannt.

Weiterhin ist es alternativ beispielsweise insbesondere auch möglich, den Herzschlag akustisch zu detektieren. Hierzu kann eine an sich bekannte Sphygmomanometer-Vorrichtung (nicht dargestellt) Verwendung finden. An einer an einer geeigneten Stelle am Körper (beispielsweise Oberarm, Handgelenk) befestigten Manschette befindet sich, über Schläuche verbunden, ein Drucksensor und ein Druckregulierer. Beim Ablassen eines zuvor aufgebauten Überdruckes treten Verwirbelungsgeräusche (Korotkow-Geräusch) auf, anhand derer mit Hilfe des Drucksensors insbesondere der systolische und diastolische arterielle Druckwert sowie der Puls ermittelt werden können.

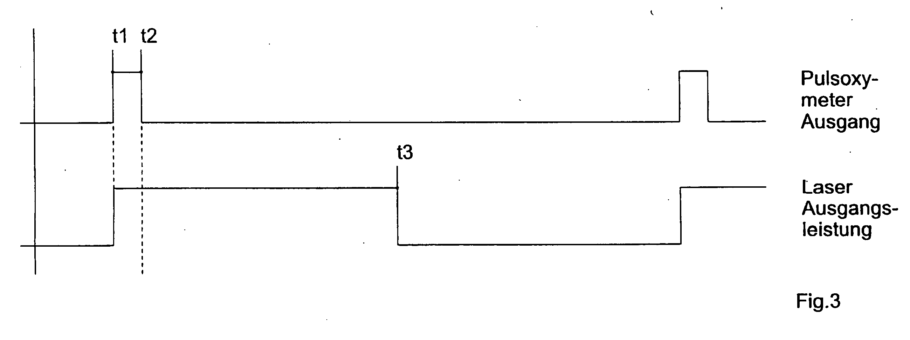

Fig. 3 zeigt ein Signaldiagramm, das die Zeitsteuerung der in den Applikatoren 101, 102 eingebauten Laserdioden veranschaulicht. Die obere Kurve zeigt schematisch den Ausgang des Pulsoxymeters 201 über die Zeit t. Zu einem ersten Zeitpunkt t1 detektiert das Pulsoxymeter 201 den Beginn der systolischen Phase des Herzzyklus und liefert an seinem Herzschlagssignal-Ausgang eine ansteigende Flanke, die zum Auslösen der in der unteren Kurve dargestellten Freigabe des Lasersignals durch die Steuerungseinrichtung 105 verwendet wird. Die Freigabe des Lasersignals durch die Steuerungseinrichtung 105 hält in einer bevorzugten Ausführungsform im wesentlichen während der Hälfte der Zeitdauer eines Herzzyklus an; in Fig. 3 bis zum Teitpunkt t3. Wenn beispielsweise die Pulsfrequenz bei der Applikation der kostmetischen Behandlung 60 Pulsschläge pro Minute beträgt, ergibt sich eine Gesamtdauer eines Herzschlagszyklus von 1 s. Bei einem Tastverhältnis von 50% des Herzschlagszyklus führt dies im vorliegenden Beispiel zu einer Zeitdauer zwischen t1 und t3 von 500 ms.

Bei einer bevorzugten Ausführungsform wird voreingestellt, dass der triggerbare Laser zu Beginn der systolischen Phase einen Laserimpuls abgibt, der eine Dauer von 25% bis 75 % des (kardiologischen) Pulsintervalles anhält. Bei einer besonders bevorzugten anderen Ausführungsform wird voreingestellt, dass der triggerbare Laser zu Beginn der systolischen Phase einen Laserimpuls abgibt, der eine Dauer von 45% bis 55% des (kardiologischen) Pulsintervalles anhält. Bei einer weiteren, darüber hinaus besonders bevorzugten anderen Ausführungsform wird voreingestellt, dass der triggerbare Laser zu Beginn der systolischen Phase einen Laserimpuls abgibt, der eine Dauer von 50% des (kardiologischen) Pulsintervalles der kosmetisch behandelten Person anhält.

Bei einer bevorzugten Ausführungsform sendet der triggerbare Laser Laserlicht im Wellenlängenbereich von 630 nm bis 790 nm aus. Bei einer besonders bevorzugten anderen Ausführungsform sendet der triggerbare Laser Laserlicht auf einer ersten Wellenlänge von 655 nm (Absorption durch Grenzwasser-Flächen) und gleichzeitig auf einer zweiten Wellenlänge von 785 nm (Absorption durch Hämoglobin zur Erhöhung des Grundumsatzes) aus.

Die erfindungsgemäße Vorrichtung wird erfindungsgemäß in einem Verfahren zur kosmetisch-ästhetischen Hautbehandlung eingesetzt. Eine Ausführungsform des erfindungsgemäßen Verfahrens umfaßt folgende Schritte:
a) Auftragen einer kurzkettigen Hyaluronsäure-Zubereitung auf einen Hautbereich. Eine Übersicht über relevante Parameter und Wirkungen von Hyaluronsäure und Hyaluronsärehaltigen Zubereitungen bei der Anwendung auf der Haut ist insbesondere bekannt aus J. Wohlrab [Hg.]: Trends in Clinical and Experimental Dermatology, Volume 3: Hyaluronsäure und Haut. Aachen: Shaker-Verlag, 2004. Für das erfindungsgemäße Verfahren ist insbesondere geeignet Hyaluronsäure-Gel (Lieferbar bei Fa. GfN Herstellung von Naturextrakten GmbH, Straßburg 16, 69483 Wald-Michelbach, Produkt Nr. 3010 (Hyaluronsäure Na-Salz)). Es handelt sich um eine kurzkettige Hyaluronsäure-Zubereitung mit biotechnologisch hergestellter Hyaluronsäure.
b) Einwirken von gepulstem Laserlicht auf den Hautbereich mittels der in Fig. 2 gezeigten und vorstehend beschriebenen erfindungsgemäßen Vorrichtung zur kosmetisch-ästhetischen Hautbehandlung, wobei das gepulste Laserlicht mit dem Herzschlag synchronisiert ist.

In einer Ausführungsform erfolgt die Synchronisation des gepulsten Laserlichtes durch ein Ausgangssignal einer elektrokardiographischen Einrichtung. In einer anderen Ausführungsform erfolgt die Synchronisation des gepulsten Laserlichtes durch ein Ausgangssignal einer Sphygmomanometer-Einrichtung. In einer anderen, bevorzugten Ausführungsform erfolgt die Synchronisation des gepulsten Laserlichtes durch ein Ausgangssignal einer Pulsoxymeter-Vorrichtung.

In einer Ausführungsform wirkt das gepulste Laserlicht durch einen Laserlicht-Impuls zu Beginn der systolischen Phase auf den Hautbereich ein. In einer bevorzugten Ausführungsform wirkt dass das gepulste Laserlicht durch einen Laserlicht-Impuls zu Beginn der systolischen Phase auf den Hautbereich während einer Dauer, die von 25% bis 75 % des (kardiologischen) Pulsintervalles beträgt, ein. In einer besonders bevorzugten Ausführungsform wirkt dass das gepulste Laserlicht durch einen Laserlicht-Impuls zu Beginn der systolischen Phase auf den Hautbereich während einer Dauer, die von 45% bis 55 % des (kardiologischen) Pulsintervalles beträgt, ein. In einer darüber hinaus besonders bevorzugten Ausführungsform wirkt dass das gepulste Laserlicht durch einen Laserlicht-Impuls zu Beginn der systolischen Phase auf den Hautbereich während einer Dauer, die 50% des (kardiologischen) Pulsintervalles beträgt, ein.

In einer Ausführungsform des Verfahrens sendet der triggerbare Laser Laserlicht im Wellenlängenbereich von 630 nm bis 790 nm aus. In einer bevorzugten Ausführungsform sendet der triggerbare Laser Laserlicht auf einer ersten Wellenlänge von 655 nm und gleichzeitig auf einer zweiten Wellenlänge von 785 nm aus.

## Patentansprüche

1. Vorrichtung zur kosmetisch-ästhetischen Hautbehandlung, aufweisend:
a) einen über einen Trigger-Eingang triggerbaren Impuls-Laser (101, 102), dessen optischer Ausgang so eingerichtet ist, dass er auf einen Hautbereich richtbar ist;
b) eine Einrichtung (105) zum Triggern des Impuls-Lasers mit einem Ausgang, der mit dem Trigger-Eingang des Impulslasers (101, 102) verbunden ist;
c) eine den Herzschlag detektierende Herzschlagsdetektor-Einrichtung (201, 202) mit einem Ausgang,
d) wobei der Ausgang der Herzschlagsdetektor-Einrichtung (201, 202) ein Herzschlag-Signal ausgibt; und
e) wobei der Ausgang der Herzschlagsdetektor-Einrichtung (201, 202) mit einem Eingang der Einrichtung (105) zum Triggern des Impuls-Lasers (101, 102) verbunden ist, so dass der triggerbare Laser (101, 102) mit dem Herzschlag synchronisierbar ist,
**dadurch gekennzeichnet,**
f) **dass** der triggerbare Impuls-Laser (101, 102) zur Abgabe eines Laserimpulses zu Beginn der systolischen Phase eingerichtet ist, der eine Dauer (t3) von 25% bis 75 % des Pulsintervalles anhält.

2. Vorrichtung nach Anspruch 1, **dadurch gekennzeichnet, dass** die Herzschlagsdetektor-Einrichtung (201, 202) ein Pulsoxymeter ist.

3. Vorrichtung nach Anspruch 1, **dadurch gekennzeichnet, dass** die Herzschlagsdetektor-Einrichtung eine elektrokardiographische Einrichtung ist.

4. Vorrichtung nach Anspruch 3, derart eingerichtet, dass der triggerbare Laser (101, 102) zu Beginn der systolischen Phase (t1) einen Laserimpuls abgibt, der eine Dauer (t3) von 45% bis 55% des Pulsintervalles anhält.

5. Vorrichtung nach einem der Ansprüche 1 bis 4, **dadurch gekennzeichnet, dass** der triggerbare Laser (101, 102) zur Aussendung von Laserlicht im Wellenlängenbereich von 630 nm bis 790 nm eingerichtet ist.

6. Vorrichtung nach Anspruch 5, **dadurch gekennzeichnet, dass** der triggerbare Laser (101, 102) zur Aussendung von Laserlicht auf einer ersten Wellenlänge von 655 nm und gleichzeitig auf einer zweiten Wellenlänge von 785 nm eingerichtet ist.

7. Verfahren zur kosmetisch-ästhetischen Hautbehandlung, umfassend folgende Schritte:
a) Auftragen einer kurzkettigen Hyaluronsäure-Zubereitung auf einen Hautbereich;
b) Einwirken von gepulstem Laserlicht auf den Hautbereich, **dadurch gekennzeichnet, daß**
c) das gepulste Laserlicht mit dem Herzschlag synchronisiert wird **dadurch gekennzeichnet, dass** das gepulste Laserlicht durch einen Laserlicht-Impuls zu Beginn der systolischen Phase (t1) auf den Hautbereich während einer Dauer (t3), die von 25% bis 75 % des Pulsintervalles beträgt, einwirkt.

8. Verfahren nach Anspruch 7, **gekennzeichnet durch** folgenden Schritt:
Synchronisation des gepulsten Laserlichtes mit einem Ausgangssignal eines Pulsoxymeters (201, 202).

9. Verfahren nach Anspruch 8, **gekennzeichnet durch** folgenden Schritt:
Synchronisation des gepulsten Laserlichtes mit einem Ausgangssignal einer elektrokardiographischen Einrichtung

10. Verfahren nach Anspruch 7, **dadurch gekennzeichnet, dass** das gepulste Laserlicht durch einen Laserlicht-Impuls zu Beginn der systolischen Phase (t1) auf den Hautbereich während einer Dauer (t3), die von 45% bis 55 % des Pulsintervalles beträgt, einwirkt.

11. Verfahren nach einem der Ansprüche 7 bis 10, **dadurch gekennzeichnet, dass** der triggerbare Laser Laserlicht (101, 102) im Wellenlängenbereich von 630 nm bis 790 nm aussendet.

12. Vorrichtung nach Anspruch 11, **dadurch gekennzeichnet, dass** der triggerbare Laser (101, 102) Laserlicht auf einer ersten Wellenlänge von 655 nm und gleichzeitig auf einer zweiten Wellenlänge von 785 nm aussendet.

13. Verwendung einer Vorrichtung nach einem der Ansprüche 1 bis 6 zur Durchführung eines Verfahrens nach einem der Ansprüche 7 bis 11.

## Claims

1. Device for cosmetic-aesthetic skin treatment, comprising:
a) a pulsed laser (101, 102) which can be triggered via a trigger input, the optical output thereof arranged in a way such that it can be directed towards a skin area,
b) means for triggering the pulsed laser, said means having an output connected with the trigger input of the pulsed laser (101, 102),
c) heartbeat detector means (201, 202) detecting the heartbeat, comprising an output,
d) the output of said heartbeat detector means (201, 202) emitting a heartbeat signal; and
e) the output of said heartbeat detector means (201, 202) connected with an input of said means (105) for triggering the pulsed laser (101, 102) in a way such that the laser which can be triggered (101, 102) can be synchronized with said heartbeat,
**characterized in that**
f) the pulsed laser (101, 102) which can be triggered is arranged for emission, at the beginning of the systolic phase, of a laser pulse lasting for a time period (t3) from 25% up to 75% of the cardiac pulse interval.

2. Device according to claim 1, **characterized in that** said heartbeat detector means (201, 202) is a cardiac pulse oxymeter.

3. Device according to claim 1, **characterized in that** said heartbeat detector means (201, 202) is an electrocardiographic means.

4. Device according to claim 3, arranged in a way such that the laser (101, 102) which can be triggered emits, at the beginning of the systolic phase (t1), a laser pulse lasting for a time period (t3) from 45% up to 55% of the cardiac pulse interval.

5. Device according to one of the claims 1 to 4, **characterized in that** the laser (101, 102) which can be triggered is arranged to emit laser light in a wavelength interval from 630 nm up to 790 nm.

6. Device according to claim 5, **characterized in that** the laser (101, 102) which can be triggered is arranged to emit laser light on a first wavelength of 655 nm and, at the same time, on a second wavelength of 785 nm.

7. Method for cosmetic-aesthetic skin treatment, comprising the following steps:
a) application of a short-chain hyaluronic acid formulation on a skin area;
b) acting of pulsed laser light on said skin area,
c) the pulsed laser light is synchronized with the heartbeat,
**characterized in that**
the pulsed laser light is acting, at the beginning of the systolic phase (t1), on said skin area during a time period (t3) from 25% up to 75% of the cardiac pulse interval.

8. Method according to claim 7, **characterized by** a step as follows:
synchronization of the pulsed laser light with an output signal of a cardiac pulse oxymeter (201, 202).

9. Method according to claim 8, **characterized by** a step as follows:
synchronization of the pulsed laser light with an output signal of an electrocardigraphic means.

10. Method according to claim 7, **characterized in that**, at the beginning of the systolic phase (t1), the pulsed laser light acts by a laser light pulse on the skin area during a time period (t3) from 45% up to 55% of the cardiac pulse interval.

11. Method according to one of claims 7 to 10, **characterized in that** the laser which can be triggered (101, 102) emits laser light within the wavelength interval from 630 nm up to 790 nm.

12. Device according to claim 1, **characterized in that** the laser which can be triggered (101, 102) emits laser light on a first wavelength of 655 nm and, at the same time, on a second wave length of 785 nm.

13. Use of a device according to one of claims 1 to 6 for execution of a method according to one of the claims 7 to 11.

## Revendications

1. Dispositif de traitement dermatologique cosmétique et esthétique, présentant :
a) un laser à impulsions (101, 102) pouvant être déclenché par une entrée de déclencheur dont la sortie optique est conçue de telle sorte qu'elle peut être orientée sur une zone cutanée,
b) un système pour déclencher le laser à impulsions doté d'une sortie qui est reliée à l'entrée de déclencheur du laser à impulsions (101, 102) ;
c) un système de détecteur de battement du coeur (201, 202) détectant le battement du coeur, doté d'une sortie,
d) dans lequel la sortie du système de détecteur de battement du coeur (201, 202) émet un signal de battement du coeur ; et
e) dans lequel la sortie du système de détecteur de battement du coeur (201, 202) est reliée à une entrée du système (105) pour déclencher le laser à impulsions (101, 102) de sorte que le laser pouvant être déclenché (101, 102) peut être synchronisé avec le battement du coeur,
**caractérisé en ce que**
f) le laser à impulsions pouvant être déclenché (101, 102) est conçu pour émettre au début de la phase systolique, une impulsion laser qui dure sur une durée (t3) de 25 % à 75 % de l'intervalle de l'impulsion.

2. Dispositif selon la revendication 1, **caractérisé en ce que** le système de détecteur de battement du coeur (201, 202) est un pulsoxymètre.

3. Dispositif selon la revendication 1, **caractérisé en ce que** le système de détecteur de battement du coeur est un système électrocardiographique.

4. Dispositif selon la revendication 3, conçu de telle sorte que le laser pouvant être déclenché (101, 102) émet au début de la phase systolique (t1) une impulsion laser qui dure sur une durée (t3) de 45 % à 55 % de l'intervalle d'impulsion.

5. Dispositif selon l'une des revendications 1 à 4, **caractérisé en ce que** le laser pouvant être déclenché (101, 102) est conçu pour émettre une lumière laser dans une plage de longueurs d'ondes de 630 nm à 790 nm.

6. Dispositif selon la revendication 5, **caractérisé en ce que** le laser pouvant être déclenché (101, 102) est conçu pour émettre une lumière laser à une première longueur d'ondes de 655 nm et en même temps à une deuxième longueur d'ondes de 785 nm.

7. Procédé de traitement dermatologique, cosmétique et esthétique, comprenant les étapes suivantes :
a) application d'une préparation d'acide hyaluronique à chaîne courte sur une zone cutanée ;
b) action de la lumière laser pulsée sur la zone cutanée,
**caractérisé en ce que**
c) la lumière laser pulsée est synchronisée avec le battement du coeur,
**caractérisé en ce que**
la lumière laser pulsée agit par une impulsion de lumière laser au début de la phase systolique (t1) sur la zone cutanée pendant une durée (t3) qui couvre de 25 % à 75 % de l'intervalle d'impulsion.

8. Procédé selon la revendication 7, **caractérisé par** l'étape suivante :
synchronisation de la lumière laser pulsée avec un signal de sortie d'un pulsoxymètre (201, 202).

9. Procédé selon la revendication 8, **caractérisé par** l'étape suivante :
synchronisation de la lumière laser pulsée avec un signal de sortie d'un système électrocardiographique.

10. Procédé selon la revendication 7, **caractérisé en ce que** la lumière laser pulsée agit par une impulsion de lumière laser du début de la phase systolique (t1) sur la zone cutanée pendant une durée (t3) qui couvre de 45 % à 55 % de l'intervalle d'impulsion.

11. Procédé selon l'une des revendications 7 à 10, **caractérisé en ce que** le laser pouvant être déclenché (101, 102) émet une lumière laser dans la plage de longueurs d'ondes de 630 nm à 790 nm.

12. Dispositif selon la revendication 1, **caractérisé en ce que** le laser pouvant être déclenché (101, 102) émet une lumière laser à une première longueur d'ondes de 655 nm et en même temps à une deuxième longueur d'ondes de 785 nm.

13. Utilisation d'un dispositif selon l'une des revendications 1 à 6, pour la réalisation d'un procédé selon l'une des revendications 7 à 11.
